# EUROPEAN PATENT APPLICATION

(11) **EP 3 837 992 A1**
(43) Date of publication of application: **23.06.2021**
(21) Application number: 19822590.6
(22) Date of filing: 12.06.2019
(51) Int. Cl.: A23L 33/135, A61K 35/744, A61P 1/16

(54) **COMPOSITION COMPRISING LEUCONOSTOC SP. STRAIN FOR IMPROVEMENT OF LIVER FUNCTION**

(30) Priority: 19.06.2018 KR 20180070289; 19.11.2018 KR 20180143027
(71) Applicant: Coenbio Co., Ltd., Seongnam-si, Gyeonggi-do 13207 (KR)
(72) Inventor: KIM, Yoon-Keun, Paju-Si, Gyeonggi-do 10908 (KR); YUM, Kyu Jin, Anyang-Si, Gyeonggi-do 14106 (KR)
(74) Representative: Danubia Patent & Law Office LLC
(86) International application number: PCT/KR2019/007069
(87) International publication number: WO 2019/245223

(57) **Abstract**

The present invention relates to a *Leuconostoc holzapfelii* strain for improvement of liver function and a use thereof. The Leuconostoc holzapfelii strain of the present invention can effectively improve functions of the liver and thus is expected to find applications in a variety of fields including various food compositions including functional food compositions, pharmaceutical compositions, and animal food compositions.

<110>    CoenBio Co.,LTD

<120>    A composition for improving liver function comprising genus
         Leuconostoc

<130>    MPCT19-055

<150>    KR 10-2018-0070289
<151>    2018-06-19

<150>    KR 10-2018-0143027
<151>    2018-11-19

<160>    2

<170>    KoPatentIn 3.0

<210>    1
<211>    50
<212>    DNA
<213>    Artificial Sequence

<220>
<223>    16S_V3_F


<400>    1
tcgtcggcag cgtcagatgt gtataagaga cagcctacgg gnggcwgcag                    50


<210>    2
<211>    55
<212>    DNA
<213>    Artificial Sequence

<220>
<223>    16S_V4_R


<400>    2
gtctcgtggg ctcggagatg tgtataagag acaggactac hvgggtatct aatcc              55

<110>    CoenBio Co.,LTD

<120>    A composition for improving liver function comprising genus
Leuconostoc

<130>    MPCT19-055

<150>    KR 10-2018-0070289
<151>    2018-06-19

<150>    KR 10-2018-0143027
<151>    2018-11-19

<160>    2

<170>    KoPatentIn 3.0

<210>    1
<211>    50
<212>    DNA
<213>    Artificial Sequence

<220>
<223>    16S_V3_F

<220>
<221>  misc_feature
<222>  (42)..(42)
<223>  n is a, c, g, or t

<400>    1
tcgtcggcag cgtcagatgt gtataagaga cagcctacgg gnggcwgcag                50

<210>    2
<211>    55
<212>    DNA
<213>    Artificial Sequence

<220>
<223>    16S_V4_R

<400>    2
gtctcgtggg ctcggagatg tgtataagag acaggactac hvgggtatct aatcc          55

## Description

### Technical Field

The present disclosure relates to a composition for improving liver function, comprising a *Leuconostoc* sp. strain, a culture broth thereof, a concentrate thereof, or a dried product thereof.

### Background Art

Liver is one of the organs that play an important role in many functions such as detoxification in the body, excretion of bile, metabolism of lipids, etc., storage of various nutrients, hematopoiesis or blood clotting, and regulation of circulating blood volume, and the reduction of the liver function causes various diseases. According to a recent report, it was confirmed that in Korea, the mortality rate for liver cancer is 23.4 per 100,000 people, which is the first in the world, and the mortality rate for a chronic liver disease is 28.8 per 100,000 people, which is third in the world. In addition, according to a report by the National Statistical Office, 56.1 per 100,000 people in their 40s in Korea have liver-related diseases, which were reported as the main cause of death in Korea. Representative liver-related diseases include fatty liver, alcoholic hepatitis, alcoholic liver cirrhosis, liver cancer, etc., and mental stress and smoking, which have recently been increased, aggravate liver damage and inhibit the detoxification effect of the human body, which can also cause other diseases (Korean patent application No. 10-2016-0103803).

As the damage caused by such liver-related diseases increases, in recent years, there is a growing interest in not only the disease treatment market, but also products for improving functions that can improve the function of the liver, and demands of consumers.

### Disclosure

### Technical Problem

The present disclosure was devised to solve the problems of the prior art as described above, and an object of the present disclosure is to provide a composition for improving liver function, comprising a *Leuconostoc* sp. strain, a culture broth thereof, a concentrate thereof, or a dried product thereof.

However, the technical problem to be achieved by the present disclosure is not limited to the problems mentioned above, and other problems that are not mentioned will be clearly understood by one skilled in the art from the following description.

### Solution to Problem

The present disclosure provides a food composition for improving liver function, comprising a *Leuconostoc holzapfelii* strain, a culture broth thereof, a concentrate thereof, or a dried product thereof.

In addition, the present disclosure provides a pharmaceutical composition for preventing or treating liver-related diseases, comprising a *Leuconostoc holzapfelii* sp. strain, a culture broth thereof, a concentrate thereof, or a dried product thereof as an active ingredient.

In an embodiment of the present disclosure, the culture broth may contain extracellular vesicles derived from *Leuconostoc holzapfelii,* which may be naturally secreted from *Leuconostoc holzapfelii,* or may be additionally added. The extracellular vesicles may be isolated from a culture broth of *Leuconostoc holzapfelii,* food containing Leuconostoc *holzapfelii,* or the supernatant obtained after killing the *Leuconostoc holzapfelii* by heating or ultrasound, etc., and may be naturally or artificially secreted, but are not limited thereto.

In another embodiment of the present disclosure, the composition may have an effect for preventing or improving the liver-related disease, and the liver-related disease is preferably a disease associated with a decrease of Streptococcus in a serum and/or a disease associated with an inflammatory liver disease, more preferably liver cancer, hepatitis, liver cirrhosis, liver failure, etc., but is not limited thereto as long as it is a disease which can be caused in the liver.

In another embodiment of the present disclosure, a daily dosage of the *Leuconostoc holzapfelii* may be preferably 5 x 10⁴ to 5 x 10¹⁰ CFU (colony forming unit)/mL, but is not limited thereto as long as it may not induce side effects by administration.

In another embodiment of the present disclosure, the composition may preferably further comprise strains such as *Leuconostoc* sp., *Lactobacillus* sp., *Enterococcus* sp., *Brevibacillus* sp., *Lactococcus* sp., *Propionibacterium* sp., *Bifidobacterium* sp., but the strains are not limited thereto as long as they are the strains known to be included in a food composition.

In yet another embodiment of the present disclosure, the composition may further comprise a cytologically acceptable carrier or a pharmaceutically acceptable carrier.

In addition, the present disclosure provides a method for treating liver-related diseases comprising: administering to an individual a composition comprising a *Leuconostoc holzapfelii* strain, a culture broth thereof, a concentrate thereof, or a dried product thereof as an active ingredient.

Further, the present disclosure provides a use of the composition for treating the liver-related diseases, comprising a *Leuconostoc holzapfelii* strain, a culture broth thereof, a concentrate thereof, or a dried product thereof as an active ingredient.

### Advantageous Effects of Invention

The present disclosure relates to a *Leuconostoc holzapfelii* strain for improving liver function and a use thereof, and the *Leuconostoc holzapfelii* strain of the present disclosure may not only effectively improve liver function without side effects, but also effectively reduce inflammation through intake, and thus has an effect for preventing, improving and/or treating various liver diseases including a disease associated with the liver inflammation. Therefore, the *Leuconostoc holzapfelii* strain of the present disclosure is expected to be applicable to various compositions such as various food compositions including functional food compositions, pharmaceutical compositions, and animal food compositions.

### Brief Description of Drawings

FIG. 1 is a view showing a serum bacterial metagenomic result of a patient with liver cirrhosis or liver cancer according to an embodiment of the present disclosure.
FIG. 2 is a view showing a result of confirming the effect of improving liver function of the *Leuconostoc holzapfelii* strain according to an embodiment of the present disclosure.
FIG. 3 is a view showing a result of confirming the effect of inhibiting the death of liver cell by *Leuconostoc holzapfelii* strain according to an embodiment of the present disclosure.
FIG. 4 is a view showing a result of confirming the presence or absence of a cytoxicity of extracellular vesicles derived from a culture broth of *Leuconostoc holzapfelii* according to an embodiment of the present disclosure.
FIG. 5 is a view showing a result of confirming whether an inflammation reaction by the extracellular vesicles derived from a culture broth of *Leuconostoc holzapfelii* according to an embodiment of the present disclosure is induced.
FIG. 6 is a view showing a result of confirming the anti-inflammatory effect of the extracellular vesicles derived from a culture broth of *Leuconostoc holzapfelii* according to an embodiment of the present disclosure.

### Best Mode for Carrying out the Invention

The *Leuconostoc holzapfelii* strain of the present disclosure may not only effectively improve liver function without side effects by inhibiting the increase of beneficial bacteria in the body and the death of liver cell through intake or administration of the strain, but also effectively inhibit the induction of inflammation through intake, and thus exhibit a preventive or therapeutic effect on liver-related diseases. Therefore, the *Leuconostoc holzapfelii* strain of the present disclosure is expected to be effectively used in various fields such as medicine, food, and feed.

In the specification of the present disclosure, *"Leuconostoc holzapfelii",* which is a morphologically gram-positive bacteria, was deposited as Leuconostoc holzapfelii Ceb-kc-003 with KCCM11830P in Korean Microorganism Deposit Center on April 11, 2016, and has 99% homology with existing *Leuconostoc holzapfelii* based on a result of 16s rDNA. The *Leuconostoc holzapfelii* of the present disclosure may be usually cultured through a method of cultivating a strain of Bacillus or *Leuconostoc* sp. strain, and as a medium, a natural medium or a synthetic medium may be used. As a carbon source of the medium, for example, glucose, sucrose, dextrin, glycerol, starch, etc. may be used, and as a nitrogen source, peptone, meat extract, yeast extract, dried yeast, soybean, ammonium salt, nitrate and other organic or inorganic nitrogen-containing compounds may be used, but the sources are not limited to thereto. As inorganic salts included in the medium, magnesium, manganese, calcium, iron, potassium, etc. may be used but the salts are not limited thereto. In addition to the components of the carbon source, nitrogen source, and inorganic salt, amino acids, vitamins, nucleic acids, and compounds related thereto may be added to the medium. The strain of the present disclosure may be cultured for 12 hours to 4 days in a temperature range of 20 to 40°C as culture temperature conditions. In addition, herbal preparations such as small black bean, Rehmannia glutinosa, licorice, cnidium officinale Makino, Eucommia Bark, cinnamon, angelica, Acorus gramineus, Polygonum multiflorum Thunberg, Platycladus orientalls, ginger, white porcelain, akane, Cimicifuga heracleifolia, Vitex rotundifolia, extracts thereof, or a mixture thereof are added and cultured, and the effect of improving liver or intestinal function of the present strain may be further improved by using this culture.

In the present specification, the term "culture broth" may be a culture stock solution containing bacterial body, and may also be bacterial body (concentrates) obtained by removing or concentrating the culture supernatant. Alternatively, the culture broth may be a culture supernatant comprising extracellular vesicles derived from *Leuconostoc* strains. The composition of the culture broth may additionally comprise a component necessary for culturing a conventional *Leuconostoc* strain, as well as a component synergistically acting on the growth of the *Leuconostoc* strain, and the composition according to this may be easily selected by one skilled in the art. The extracellular vesicles refer to a structure made of a nano-sized membrane secreted from various bacteria, and may be isolated from a culture broth of *Leuconostoc* strain or a food comprising *Leuconostoc* strain in the present invention, or may be artificially secreted, but is not limited thereto. The method of separating the extracellular vesicles from the culture broth or food is not particularly limited as long as the extracellular vesicles may be separated. Extracellular vesicles may be separated by using methods such as centrifugation, ultra-high-speed centrifugation, filtration by filter, gel filtration chromatography, pre-flow electrophoresis, or capillary electrophoresis, and combinations thereof, and the method may further comprise a process such as washing for removal of the impurities and concentration of the obtained extracellular vesicles.

In the present specification, the term "composition" may be a medicine, food, animal food, pharmaceutical, beverage, lactic acid bacterium preparation, etc. comprising *Leuconostoc holzapfelii* as an active ingredient, and is not limited thereto as loan as it is a composition comprising *Leuconostoc holzapfelii* strain. The pharmaceutical composition may be a quasi-drug or a pharmaceutical preparation, and the food composition may be a food, a health food, a health supplement, or a health functional food, but is not limited thereto.

In addition, the state of the strain may be a liquid state or a dry state, and the drying method may be air drying, natural drying, spray drying, freeze drying, etc., but is not limited thereto.

In the present specification, "prevention" refers to any action that inhibits or delays onset of diseases such as liver-related diseases by administration of the composition according to the present invention.

In the present specification, "treatment" refers to any action in which symptoms of liver-related diseases, etc. are improved or beneficially changed by administration of the composition according to the present invention.

In the present specification, "improvement" refers to any action that at least reduces the severity of a parameter related to the condition being treated, for example, symptoms.

In the present specification, an "individual" refers to a subject in need of prevention or treatment of a disease, and specifically refers to a mammal such as a human or non-human primate, mouse, rat, dog, cat, horses, and cows.

In the present disclosure, the food composition may be used in various foods, such as beverages, gums, teas, vitamin complexes, lactic acid bacteria complexes, health supplement foods, functional foods, etc., and may be used in the form of pills, powders, granules, infusions, tablets, capsules or beverages. At this time, the amount of the *Leuconostoc holzapfelii* strain, a culture broththereof, a concentrate thereof, or a dried product thereof in a food or beverage may be generally added in an amount of 0.01 to 15% by weight of the total food weight in the case of the food composition of the present disclosure, and may be added in a ratio of 0.02 to 10 g, preferably 0.3 to 1 g, based on 100 mL in the case of a healthy beverage composition.

The food composition of the present disclosure may include conventional food additives in the art, such as flavoring agents, taste agents, coloring agents, fillers, stabilizers, etc. The food composition according to the present disclosure may comprise various flavoring agents or natural carbohydrates, etc. as an additional component, like ordinary foods, without any particular limitation on the component to be added, in addition to the *Leuconostoc holzapfelkii* strain, a culture broththereof, a concentrate thereof, or a dried product thereof as an essential ingredient. Examples of the natural carbohydrate include conventional sugar, for example, monosaccharides such as glucose, fructose, etc.; disaccharides such as maltose, sucrose, etc.; polysaccharides such as dextrin, cyclodextrin, etc.; and sugar alcohols such as xylitol, sorbitol, erythritol, etc. As flavoring agents other than those described above, natural flavoring agents (taumatin, stevia extract (e.g., rebaudioside A, glycyrrhizin, etc.)) and synthetic flavoring agents (saccharin, aspartame, etc.) may be advantageously used. The proportion of the natural carbohydrate is generally about 1 to 20 g, preferably about 5 to 12 g per 100 mL of the composition of the present disclosure.

In addition to those described above, the food composition of the present disclosure may comprise various nutrients, vitamins, minerals (electrolytes), flavoring agents such as synthetic flavors and natural flavoring agents, coloring agents and thickeners (cheese, chocolate, etc.), pectic acid and a salt thereof, alginic acid, and a salt thereof, organic acids, protective colloidal thickeners, pH adjusters, stabilizers, preservatives, glycerin, alcohols, carbonates used in carbonated beverages, etc. These components may be used independently or in combination. The proportion of these additives is not so important, but is generally selected from the range of 0 to about 20 parts by weight per 100 parts by weight of the composition of the present disclosure.

In the present specification, the term "pharmaceutical composition" may be characterized in the form of capsules, tablets, granules, injections, ointments, powders, or beverages, and the pharmaceutical composition is applied to humans as a subject. The pharmaceutical composition is not limited to thereto, but may be used in a formulation of oral dosage forms such as powders, granules, capsules, tablets, aqueous suspensions, external preparations, suppositories and sterile injectable solutions according to a conventional method. The pharmaceutical composition of the present disclosure may comprise a pharmaceutically acceptable carrier. As a pharmaceutically acceptable carrier, in the case of oral administration, binders, lubricants, disintegrants, excipients, solubilizers, dispersants, stabilizers, suspending agents, colors, flavors, etc. may be used, in the case of injection, buffering agents, preservatives, painlessness agents, solubilizers, isotonic agents, stabilizers, etc. may be mixed and used, and in the case of topical administration, base agents, excipients, lubricants, preservatives, etc. may be used. The formulation of the pharmaceutical composition of the present disclosure may be variously prepared by mixing with a pharmaceutically acceptable carrier as described above. For example, in the case of oral administration, the pharmaceutical composition of the present disclosure may be prepared in the form of tablets, troches, capsules, elixir, suspension, syrup, wafers, etc., and in the case of injections, the pharmaceutical composition of the present disclosure may be prepared in unit dosage ampoules or multiple dosage forms. In addition, the pharmaceutical composition of the present disclosure may be formulated as solutions, suspensions, tablets, capsules, sustained-release preparations, etc.

Meanwhile, examples of carriers, excipients and diluents suitable for the formulation may include lactose, dextrose, sucrose, sorbitol, mannitol, xylitol, erythritol, malditol, starch, gum acacia, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate or mineral oil. In addition, examples thereof may further include fillers, anti-aggregating agents, lubricants, wetting agents, flavoring agents, emulsifying agents, preservatives, etc.

The route of administration of the pharmaceutical composition according to the present disclosure incudes, but is not limited to, oral, intravenous, intramuscular, intraarterial, intramedullary, intrathecal, intracardiac, transdermal, subcutaneous, intraperitoneal, intranasal, intestinal, local, sublingual or rectal administration. Oral or parenteral administration is preferred. The term "parenteral" used herein includes subcutaneous, intradermal, intravenous, intramuscular, intraarticular, intrasynovial, intrasternal, intrathecal, intralesional and intracranial injection or infusion techniques. The pharmaceutical composition of the present disclosure may also be administered in the form of suppositories for rectal administration.

The pharmaceutical composition of the present disclosure may vary in various ways, depending on a number of factors including the activity of the specific compound used, age, weight, general health, sex, formulation, time of administration, route of administration, excretion rate, drug combination, and the severity of the specific disease to be prevented or treated, and the dosage of the pharmaceutical composition varies depending on the patient's condition, weight, degree of disease, drug type, administration route and duration, but may be appropriately selected by one skilled in the art, and be administered at 0.0001 to 50 mg /kg or 0.001 to 50 mg/kg per day. The administration may be carried out once a day, or may be divided several times. The above dosage does not in any way limit the scope of the present disclosure. The pharmaceutical composition according to the present disclosure may be formulated as pills, dragees, capsules, liquids, gels, syrups, slurries, or suspensions.

The dosage or intake of the *Leuconostoc holzapfelii* of the present disclosure is, for example, 5 x 10⁴ to 5 x 10⁸ CFU/mL of the *Leuconostoc holzapfelii* Ceb-kc-003 (KCCM11830P) strain, preferably 1 x 10⁶ to 1 x 10⁸ CFU/ml of the *Leuconostoc holzapfelii* Ceb-kc-003 (KCCM11830P), and may be administered once to 4 times a day in 30 mL to 100 mL. For example, 50 mL per dose may be administered 2 to 4 times a day in an amount of 1 x 10⁶ to 1 x 10⁸ CFU/ml of *Leuconostoc holzapfelii* Ceb-kc-003 (KCCM11830P). More specifically, 50 mL to 100 mL per dose may be administered 1 to 2 times a day in an amount of 1 x 10⁶ to 1 x 10⁸ CFU/ml of *Leuconostoc holzapfelii* Ceb-kc-003 (KCCM11830P). Still more specifically, 1 x 10⁶ to 1 x 10⁸ CFU/mL of *Leuconostoc holzapfelii* Ceb-kc-003 (KCCM11830P) may be administered once in 50 mL to 100 mL 30 minutes before breakfast, and once in 50 mL to 100 mL 30 minutes before dinner or before bedtime. However, this dosage or intake may vary depending on the individual's weight, age, sex, health condition, major symptoms to be treated, prevented, or improved, administration time, administration method, severity, etc.

Hereinafter, the following examples are presented to aid in understanding the present disclosure. However, the following examples are only provided for easier understanding of the present disclosure, and the contents of the present disclosure are not limited by the following examples.

### Examples

### Example 1: Serum bacterial metagenomic analysis of patients with liver-related diseases

For metagenomic analysis of bacteria in the serum of patients with liver-related diseases, serum was obtained from 99 patients with liver cirrhosis and 94 patients with liver cancer. Serum of 99 normal subjects was used as a control group. In order to separate bacterial-derived extracellular vesicles from serum and separate DNA from extracellular vesicles, the obtained serum was placed in a 10 mL tube and centrifuged at 3,500 x g at 4°C for 10 minutes to separate a pellet and a supernatant, and then the separated pellet and supernatant were added to each new 10 mL tube. After removing bacteria and foreign substances using a 0.22 µm filter, the supernatant was transferred to a centrifugal filter 50 kD and centrifuged at 1,500 X g for 15 minutes at 4°C to remove substances smaller than 50 kD and then concentrated to a total volume of 10 mL. Then, after cleanly removing bacteria and foreign substances by using a 0.22 µm filter again, ultra-high-speed centrifugation was carried out for 3 hours at 150,000 X g at 4°C using a Type 90ti rotor. After ultra-high-speed centrifugation, the supernatant was removed and the pellet was resuspended in phosphate buffered saline (PBS) and stored. Thereafter, 100 µL of the resuspended pellet was added to a new tube and heated in a 100°C heat block so that the DNA inside the extracellular vesicles was released outside the lipid. Then, after cooling with ice, centrifugation at 4°C at 10,000 X g for 30 minutes was carried out to separate only the supernatant, and then the amount of DNA in the supernatant using Nanodrop was quantified. In order to confirm the presence of bacterial-derived DNA in the DNA isolated from the serum, a polymerase chain reaction was carried out using the 16s rDNA primer shown in Table 1 to amplify the DNA.

**Table 1**

| **Primer** | | **Sequence** | **Sequence Number** |
|---|---|---|---|
| **16S rDNA** | **16S_V3_F** | 5'-TCGTCGGCAGCGTCAGATGTGTATAAGAGACAGCCTACG GGNGGCWGCAG-3' | **1** |
| | **16S_V4_R** | 5'-GTCTCGTGGGCTCGGAGATGTGTATAAGAGACAGGACTA CHVGGGTATCTAATCC-3 | **2** |

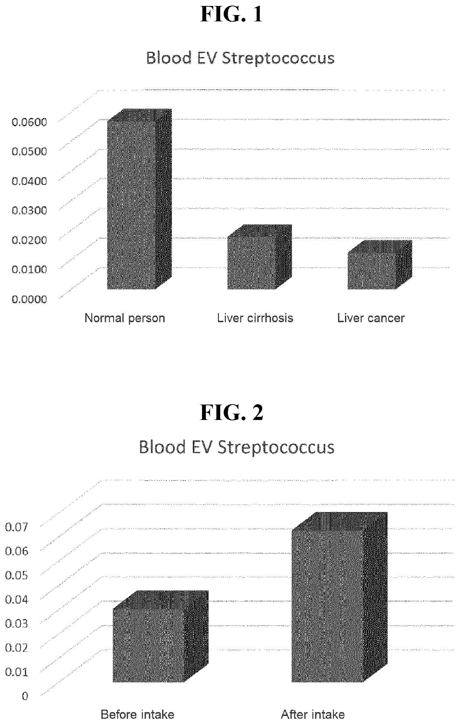

The amplified DNA was subjected to nucleotide sequence analysis using an Illumina MiSeq sequencer, and the result was output as a Standard Flowgram Format (SFF) file. The output SFF file was converted into a nucleotide sequence file (fasta) and a nucleotide quality score file using GS FLX software (v2.9) to confirm the credit rating of the read. Excluding the portion with a window 20 bps average base call accuracy of less than 99% (Phred quality score < 20), only the read length of 300 bps or more was used (Sickle version 1.33). For the result analysis, clustering according to sequence similarity was carried out using UCLUST and USEARCH, and bacteria with a nucleotide sequence similarity of 97% or more was analyzed (QIIME) using 16s rDNA nucleotide sequence database (108,453 nucleotide sequence) of BLASTN and GreenGenes, and then the operational taxonomy unit (OTU) was analyzed. For statistical analysis, a t-test was used, and bacteria present in significantly different ratios in the control group and the experimental group were selected as the case where the average distribution ratio of each group was 2 or more times different and the p value was 0.05 or less. The results are shown in FIG. 1 and Table 2.

**Table 2**

| | **Normal person** | | **Liver cirrhosis** | | | **Liver cancer** | | |
|---|---|---|---|---|---|---|---|---|
| | **mean** | **SD** | **mean** | **SD** | ***p*** | **mean** | **SD** | ***p*** |
| **Streptococcus** | 0.0568 | 0.0553 | 0.0177 | <0.05 | 0.0000 | 0.0124 | 0.0124 | < 0.0001 |

As shown in FIG. 1 and Table 2, it was confirmed that Streptococcus bacteria were significantly reduced in patients with liver-related diseases, that is, patients with liver cirrhosis and liver cancer compared to the control group. Based on the above results, it could be confirmed that liver-related diseases may be diagnosed through the reduction of Streptococcal bacteria in the serum.

### Example 2: Preparation of powder and culture broth of Leuconostoc holzapfelii strain

In order to confirm the ability to improve the intestinal or liver function of the *Leuconostoc holzapfelii* strain, the *Leuconostock holzapfelii* strain Ceb-kc-003 (KCCM11830P) strain deposited in the Korea Microorganism Deposit Center was obtained and cultured in BHI solid medium to which 3% sodium chloride was added. The powder of *Leuconostoc holzapfelii* was prepared by lyophilizing the cultured strain, and in order to prepare a culture broth, 2 kg of dextrose, 1.5 kg of whole milk powder, and 0.05 kg of yeast extract were added to 100 L of purified water, sterilized at high temperature and high pressure for 15 to 30 minutes at 121°C, and then cooled at room temperature. Also, 0.2 to 0.4 L of *Leuconostoc holzapfelii* Ceb-kc-003 strain was aseptically inoculated, and then the culture broth of *Leuconostoc holzapfelii* was prepared by culturing at about 35°C for 2 to 3 days.

### Example 3: Confirmation of effect of improving liver function of Leuconostoc holzapfelii strain

In order to confirm the effect of improving liver function of the *Leuconostoc holzapfelii* strain, a total of 20 volunteers (8 men and 12 women) were recruited, and the average age of the volunteers was 32.0 ± 5.4 years, and the average age of men was 31.8. ± 3.6 years (26 to 37 years old), and the average age of women was 32.2 ± 6.3 years (21 to 47 years old). The culture broth of the *Leuconostoc holzapfelii* Ceb-kc-003 strain was administered orally twice a day for a total of 4 weeks to a subject so that the *Leuconostoc holzapfelii* Ceb-kc-003 was 1 x 10⁶ to 1 x 10⁸ CFU (colony forming unit)/mL. Also, in the same manner as in Example 1, the volunteer's serum was collected on the day of the start of the clinical trial (before intake) and 4 weeks after (after intake), and changes in Streptococcus bacteria contained in the serum were confirmed. The results are shown in FIG. 2 and Table 3.

**Table 3**

| | **Before intake** | | **After intake** | | ***P* value** |
|---|---|---|---|---|---|
| **Streptococcus** | 0.0302 | 0.0332 | 0.0626 | 0.0396 | <0.05 |

As shown in FIG. 2 and Table 3, it was confirmed that the number of Streptococcus, a biomarker of liver cirrhosis and liver cancer, was significantly increased after intake of the *Leuconostoc* strain. Based on the above results, it could be confirmed that it is possible to inhibit the induction of liver diseases such as liver cirrhosis and liver cancer through intake of the *Leuconostoc* strain, as well as to prevent, improve and/or inhibit symptoms of liver-related diseases through the increase of beneficial bacteria in the body.

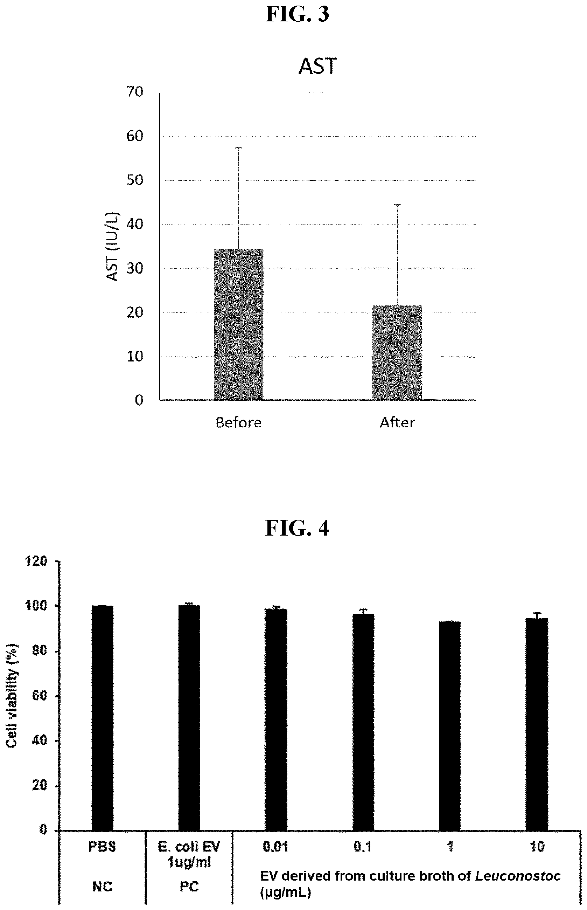

In addition, in order to further confirm the effect of improving liver function, the level of aspartate aminotransferase (AST), an index of liver-related diseases in the blood, was confirmed. Aspartate aminotransferase is present in hepatocytes, and when the cells are necrotized, they are leaked into the blood, and the level in the blood is increased. The results are shown in FIG. 3.

As shown in FIG. 3, it was confirmed that the level of aspartate aminotransferase was 34.4 ± 22.8 IU/L before intake, but significantly decreased to 21.7 ± 8.2 IU/L after intake. Based on the above results, it could be confirmed that the death of liver cell may be reduced through intake of the *Leuconostoc* strain, thereby preventing liver-related diseases.

Also, in addition to the *Leuconostoc holzapfelii* strain, a mixed strain culture broth comprising *Leuconostoc mecenteroides* KCCM11827P, *Lactobacillus sakei* KCCM11841P, *Enterococcus pecium* KCCM11909P, *Brevibacillus reuszeri* KCCM11911P, and *Lactobacillus fermentum* KCCM11910P was orally administered to 20 volunteers twice a day for 4 weeks, and changes in bacteria included in the serum were confirmed. As a result, it could be confirmed that the number of Streptococcus in the serum was further increased, compared to the case of administration of the *Leuconostoc holzapfelii* strain alone.

### Example 4: Confirmation of anti-inflammatory effect of extracellular vesicles derived from culture broth of Leuconostoc holzapfelii

### 4.1. Confirmation of cytotoxicity of extracellular vesicles derived from culture broth of Leuconostoc holzapfelii

In order to confirm the cytotoxicity of the extracellular vesicles derived from the culture broth of *Leuconostoc holzapfelii,* a culture broth of *Leuconostoc holzapfelii* Ceb-kc-003 strain was prepared in the same manner as in Example 2. In addition, a supernatant wherein cells were removed from the culture broth was obtained using a Bottle Top Vacuum Filter (Corning) having a pore size of 0.45 µm. The obtained supernatant was again passed through a 0.22 µm Bottle Top Vacuum Filter (Corning) to remove the residual cells remaining in the supernatant, and the extracellular vesicles derived from the culture broth of *Leuconostoc holzapfelii* were separated. Separated extracellular vesicles were prepared at concentrations of 0.01, 0.1, 1, and 10 µg/mL using a phosphate buffer solution, treated with peritoneal macrophages (Raw264.7) of mice, cultured for 12 hours, and then cell viability (cell viability) was measured. After staining the cultured cells with trypan blue, the number of viable cells was measured using a Neubauer chamber, and the cell viability was calculated as a percentage of the number of viable cells of the negative control. As a negative control (NC) a phosphate buffer solution was added and cultured, and as a positive control (PC), 1 µg/mL of extracellular vesicles (E. Coli EV) derived from Escherichia coli (E. coli) were added and cultured. In order to isolate the extracellular vesicles, E. coli was inoculated into Luria-Bertani (LB) medium and cultured at 37°C at 200 rpm for 24 hours. In addition, a supernatant from which cells were removed was obtained using a Bottle Top Vacuum Filter (Corning) having a pore size of 0.45 µm. The obtained supernatant was again passed through a 0.22 µm Bottle Top Vacuum Filter (Corning) to remove cell residues remaining in the supernatant, and extracellular vesicles of E. coli were prepared by separating them. The results are shown in FIG. 4.

As shown in FIG. 4, it was confirmed that the extracellular vesicles derived from the culture broth of *Leuconostoc holzapfelii* did not show cytotoxicity even at high concentrations, and thus may be used stably.

### 4.2. Confirmation of induction of inflammatory response in extracellular vesicles derived from Leuconostoc holzapfelii

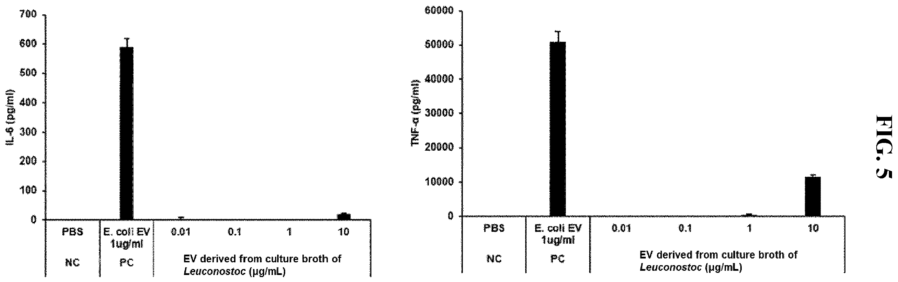

In order to confirm whether the extracellular vesicles derived from the culture broth of *Leuconostoc holzapfelii* induces the inflammatory response of the cells, the extracellular vesicles derived from the culture brothof *Leuconostoc holzapfelii* prepared in the same manner as in Example 4.1 were prepared at a concentration of 0.01, 0.1, 1, and 10 µg/mL using a phosphate buffer solution, treated with Raw264.7 cell line, incubated for 12 hours, then the secretion amount of inflammatory cytokines IL-6 and TNF-alpha was confirmed with a measurement using an enzyme-linked immunosorbent assay (ELISA). As a negative control, a phosphate buffer solution was added and cultured, and as a positive control, 1 µg/mL of E. coli-derived extracellular vesicles (E. coli EV) was added and cultured. The results are shown in FIG. 5.

As shown in FIG. 5, it was confirmed that extracellular vesicles derived from E. coli as the positive control significantly increased the amount of the secretion of inflammatory cytokines in the Raw264.7 cell line, but the extracellular vesicles derived from the culture broth of *Leuconostoc holzapfelii* only slightly increased when treated with a high concentration of 10 µg/mL.

### 4.3. Confirmation of anti-inflammatory effect of extracellular vesicles derived from Leuconostoc holzapfelii

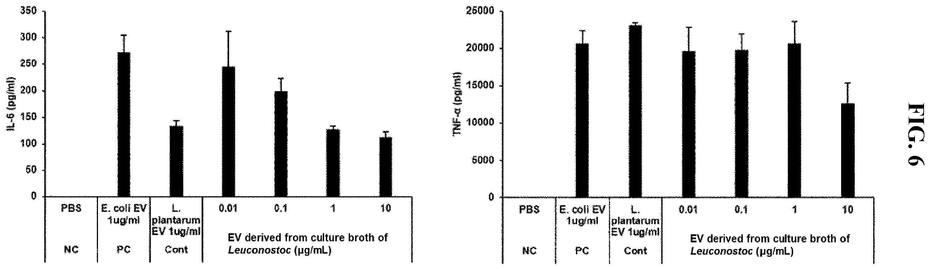

In order to confirm the anti-inflammatory effect of the extracellular vesicles derived from the culture broth of *Leuconostoc holzapfelii,* the extracellular vesicles derived from the culture broth of *Leuconostoc holzapfelii* prepared in the same manner as in Example 4.1 were prepared at a concentration of 0.01, 0.1, 1, and 10 µg/mL using a phosphate buffer solution, and treated with Raw264.7 cell line, incubated for 12 hours, and then E. coli-derived extracellular vesicles were additionally treated at a concentration of 1 µg/mL, cultured for 6 hours, and the secretion amount of IL-6 and TNF-α was confirmed with a measurement using an ELISA. As a control group (Cont), the experiment was conducted in the same manner using 1 µg/mL of extracellular vesicles derived from *Lactococcus plantarum,* which is known to exhibit existing anti-inflammatory effects. The results are shown in FIG. 6.

As shown in FIG. 6, it was confirmed that the pretreatment of the extracellular vesicles derived from the culture broth of *Leuconostoc holzapfelii* may effectively prevent and inhibit the inflammatory reaction.

Based on the above results, it could be confirmed that the *Leuconostoc holzapfelii* strain of the present disclosure does not exhibit cytotoxicity, so it can be used stably, and can effectively prevent or inhibit the induction of related diseases through the effect of improving liver function. In addition, it could be confirmed that the intake of *Leuconostoc holzapfelii* strain, culture broth, or extracellular vesicles derived from culture broth may effectively prevent the induction of liver inflammation, and thus significantly reduce the incidence of inflammatory liver diseases such as hepatitis, liver cirrhosis, and liver cancer. In addition, it could be confirmed that a more improved effect can be obtained through mixing of substances, compounds, strains, etc. having other known efficacy, in addition to the *Leuconostoc holzapfelii* strain.

The description of the present disclosure described above is for illustrative purposes only, and one skilled in the art to which the present disclosure pertains will understand that it is possible to easily transform it into other specific forms without changing the technical spirit or essential features of the present disclosure. Therefore, it should be understood that the embodiments described above are illustrative in all respects and not limiting.

### Industrial Applicability

The present disclosure relates to the *Leuconostoc holzapfelii* strain for improving liver function and a use thereof, and since the *Leuconostoc holzapfelii* strain of the present disclosure can effectively improve liver function without side effects, and also effectively reduce the inflammation through intake, it can be broadly used for preventing, improving and/or treating various liver diseases including diseases associated with the liver inflammation.

## Claims

1. A food composition for improving liver function, comprising a *Leuconostoc holzapfelii* strain, a culture broth thereof, a concentrate thereof, or a dried product thereof.

2. The food composition according to claim 1, wherein the culture broth comprises extracellular vesicles derived from *Leuconostoc holzapfelii.*

3. The food composition according to claim 1, wherein the composition has an effect of preventing a liver-related disease.

4. The food composition according to claim 3, wherein the liver-related disease is a disease associated with a decrease of Streptococcus bacteria in a serum, or a disease associated with an inflammatory liver disease.

5. The food composition according to claim 4, wherein the disease is hepatitis, liver cancer, liver cirrhosis, or liver failure.

6. The food composition according to claim 1, further comprising a strain of *Leuconostoc* sp., *Lactobacillus* sp., *Enterococcus* sp., *Brevibacillus* sp., *Lactococcus* sp., *Propionibacterium* sp., or *Bifidobacterium* sp.

7. A pharmaceutical composition for preventing or treating a liver-related disease, comprising a *Leuconostoc holzapfelii* strain, a culture broth thereof, a concentrate thereof, or a dried product thereof.

8. The pharmaceutical composition according to claim 7, wherein the culture broth comprises extracellular vesicles derived from *Leuconostoc holzapfelii.*

9. The pharmaceutical composition according to claim 7, wherein the liver-related disease is a disease associated with a decrease of Streptococcus bacteria in a serum, or a disease associated with an inflammatory liver disease.

10. The pharmaceutical composition according to claim 9, wherein the disease is hepatitis, liver cancer, liver cirrhosis, or liver failure.

11. A method for preventing, improving, or treating a liver-related disease, comprising: administering to an individual a composition comprising a *Leuconostoc holzapfelii* strain, a culture broth thereof, a concentrate thereof, or a dried product thereof as an active ingredient.

12. A use of a composition for preventing, improving, or treating liver-related disease, comprising a *Leuconostoc holzapfelii* strain, a culture broth thereof, a concentrate thereof, or a dried product thereof as an active ingredient.
